# EUROPÄISCHE PATENTSCHRIFT

(11) **EP 3 763 722 B1**
(45) Veröffentlichungstag und Bekanntmachung des Hinweises auf die Patenterteilung: **07.09.2022**
(21) Anmeldenummer: 19185648.3
(22) Anmeldetag: 11.07.2019
(51) Int. Cl.: C07F 9/6574, C07C 45/50, C07C 47/02

(54) **BISPHOSPHITE MIT EINEM OFFENEN UND EINEM GESCHLOSSENEN FLÜGELBAUSTEIN**
BIS-PHOSPHITES WITH AN OPEN AND A CLOSED BLADE MODULE
BISPHOSPHITES COMPORTANT UN COMPOSANT PÉRIPHÉRIQUE FERMÉ ET UN COMPOSANT PÉRIPHÉRIQUE OUVERT

(43) Veröffentlichungstag der Anmeldung: 13.01.2021
(73) Patentinhaber: Evonik Operations GmbH, 45128 Essen (DE)
(72) Erfinder: SALE, Anna Chiara, 45657 Recklinghausen (DE); FRANCKE, Robert, 45772 Marl (DE); BRÄCHER, Alexander, 45721 Haltern am See (DE); FRIDAG, Dirk, 45721 Haltern am See (DE); QUELL, Thomas, 2030 Antwerpen (BE)
(74) Vertreter: Evonik Patent Association

(56) Entgegenhaltungen:
- EP-A1- 0 556 681
- EP-A2- 0 213 639
- WO-A1-2008/115740
- PACIELLO ROCCO ET AL: "Structure-activity relationship for chelating phosphite ligands used in rhodium-catalyzed hydroformylations", JOURNAL OF MOLECULAR CATALYSIS A: CHEMICAL, ELSEVIER, AMSTERDAM, NL, Bd. 143, Nr. 1-3, 1. Januar 1999 (1999-01-01), Seiten 85-97, XP002499694, ISSN: 1381-1169, DOI: 10.1016/S1381-1169(98)00370-7
- DATABASE CAPLUS [Online] CHEMICAL ABSTRACTS SERVICE, COLUMBUS, OHIO, US; 1995, XP002796589, gefunden im STN Database accession no. 1995:557183

## Beschreibung

Die Erfindung betrifft Bisphosphite, die einen offenen und einen geschlossenen Flügelbaustein aufweisen. Einer der beiden Flügelbausteine weist also eine C-C-Brücke zwischen den beiden Phenylresten auf, der andere der beiden Flügelbausteine nicht.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

In A. van Rooy et al., "Bulky Diphosphite-Modified Rhodium Catalysts: Hydroformylation and Characterization", Organometallics, 1996, 15(2), Seiten 835-847, werden unterschiedliche Liganden zur Hydroformylierung beschrieben. Hierunter auch der folgende Ligand:

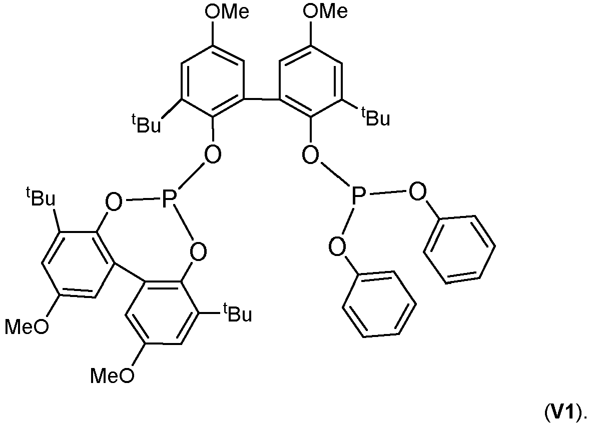

In EP 0213639 A2 werden Bisphosphitliganden und ihre Verwendung in Übergangsmetallkomplex-katalysierte Reaktionen beschrieben. Insbesondere werden Bisphosphitliganden mit sowohl einer Diorganophosphitfunktionalität als auch einer Triorganophosphitfunktionalität und deren Verwendung beschrieben.

Die technische Aufgabe der Erfindung ist die Bereitstellung neuer Liganden, welche in der Hydroformylierung von Olefinen eine gesteigerte n-Regioselektivität verglichen zu dem aus dem Stand der Technik bekennten Liganden aufweisen.

Die Aufgabe wird gelöst durch eine Verbindung gemäß Anspruch 1.

Verbindung der Formel (**I**): 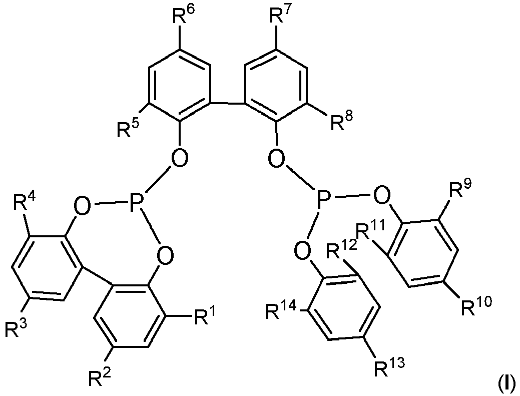 wobei
R¹, R⁴, R⁵, R⁸, R⁹, R¹¹, R¹², R¹⁴ ausgewählt sind aus: -H, -(C₁-C₁₂)-Alkyl, -O-(C₁-C₁₂)-Alkyl, Halogen, -NH₂, -NO₂;
R², R³, R⁶, R⁷ ausgewählt sind aus: -H, -O-(C₁-C₁₂)-Alkyl, -NH₂, -NO₂;
R¹⁰, R¹³ für -NO₂ stehen.

In einer Ausführungsform sind R¹, R⁴, R⁵, R⁸, R⁹, R¹¹, R¹², R¹⁴ ausgewählt aus: -H, -(C₁-C₁₂)-Alkyl, -O-(C₁-C₁₂)-Alkyl, -NO₂.

In einer Ausführungsform stehen R² und R³ für -O-(C₁-C₁₂)-Alkyl.

In einer Ausführungsform stehen R² und R³ für -O-CH₃.

In einer Ausführungsform stehen R⁶ und R⁷ für -O-(C₁-C₁₂)-Alkyl.

In einer Ausführungsform stehen R⁶ und R⁷ für -O-CH₃.

In einer Ausführungsform stehen R¹ und R⁴ für -(C₁-C₁₂)-Alkyl.

In einer Ausführungsform stehen R¹ und R⁴ für -^{t}Bu.

In einer Ausführungsform stehen R⁵ und R⁸ für -(C₁-C₁₂)-Alkyl.

In einer Ausführungsform stehen R⁵ und R⁸ für -^{t}Bu.

In einer Ausführungsform stehen R⁹, R¹¹, R¹², R¹⁴ für -H.

In einer Ausführungsform weist die Verbindung die Struktur (3) auf:

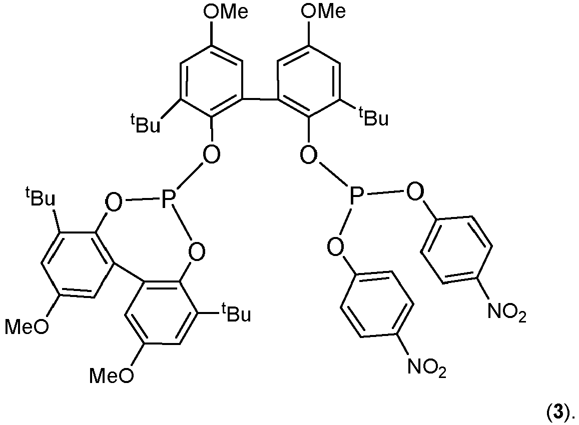

Neben der Verbindung als solche wird auch deren Verwendung zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird ein Verfahren beansprucht, in welchem die zuvor beschriebene Verbindung als Ligand eingesetzt wird.

Verfahren umfassend die Verfahrensschritte:
a) Vorlegen eines Olefins,
b) Zugabe einer zuvor beschriebenen Verbindung und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

In einer bevorzugten Ausführungsform ist das Metall Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 10 bis 70 bar.

Besonders bevorzugt sind eine Temperatur von 100 °C bis 140 °C und ein Druck von 40 bis 60 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei derTetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden α-Olefine, endständig verzweigte, innenständige und innenständig verzweigte Olefine hydroformyliert werden.

Im Folgenden soll die Erfindung anhand von Ausführungsbeispielen näher erläutert werden.

### Arbeitsvorschriften

### Allgemeine Analytik

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet.

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR³¹P = SR¹H * (BF³¹P / BF¹H) = SR¹H * 0,4048.

### Allgemeine Synthese

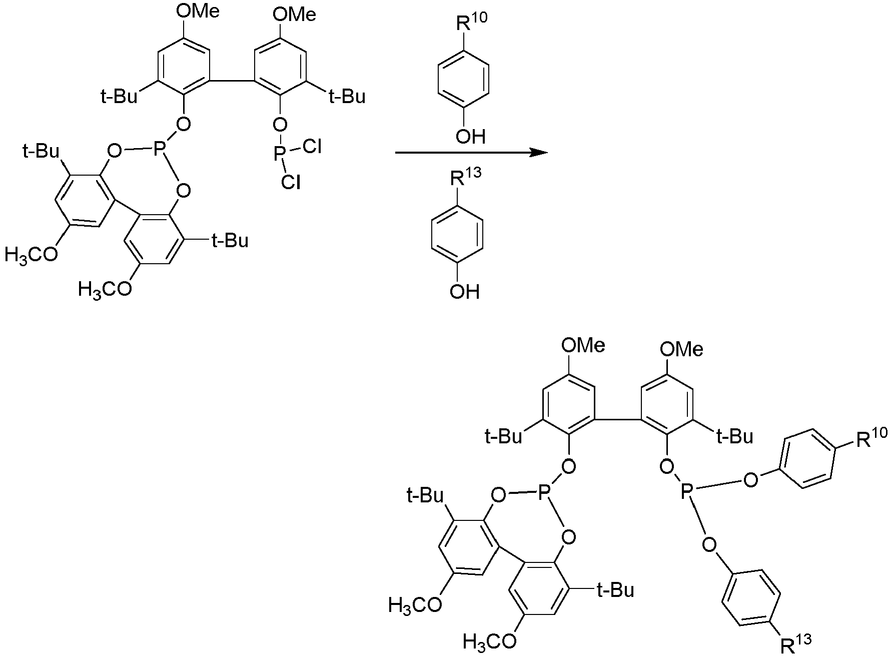

In einem sekurierten Schlenk wurde das entsprechend substituierte Phenol abgewogen und über Nacht mittels Ölpumpenvakuum bei Raumtemperatur nachgetrocknet. Am nächsten Morgen wurde unter Rühren Toluol und entgastes Triethylamin bei Raumtemperatur zugegeben. Zu dieser Phenol-Triethylamin-Lösung wurde dann langsam und stetig bei Raumtemperatur das Dichlorophosphit hinzugegeben. Die Temperatur wurde hierbei langsam auf 60 °C angehoben. Anschließend wurde das Reaktionsgemisch auf 70 °C gebracht und 2 Tage lang gerührt. Das erhaltene Produkt wurde gewaschen und gefrittet.

Reinheit: 92 bis 96%, Ausbeute: 70 bis 92%.

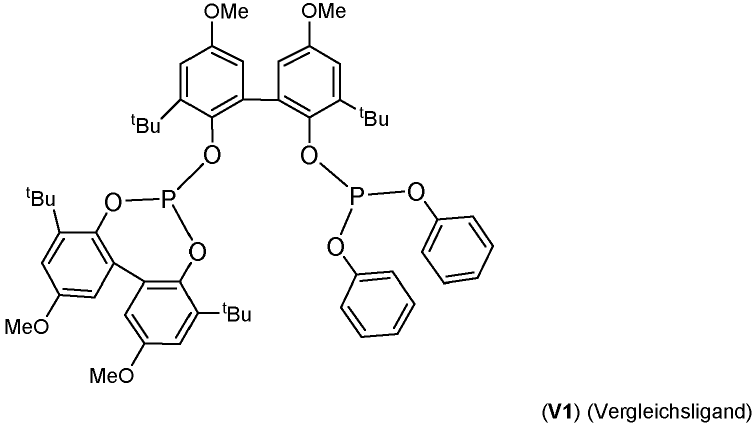

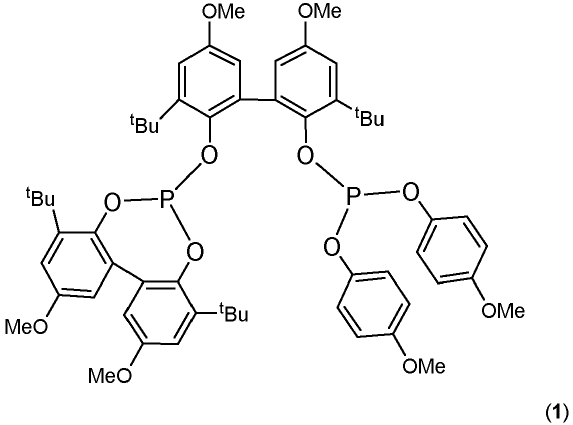

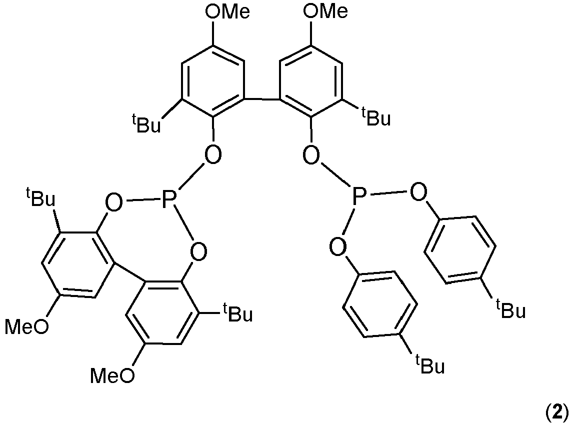

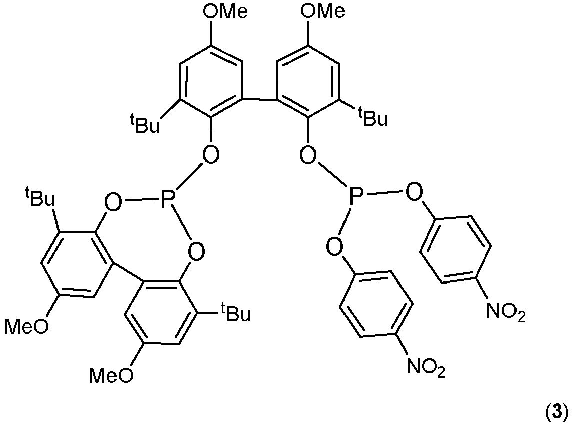

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzte Gemisch der n-Octene (Oxeno GmbH, Octenisomerengemisch aus 1-Octen: 3 %; cis+trans-2-Octen: 49 %; cis+trans-3-Octen: 29 %; cis+trans-Octen-4: 16 %; gerüstisomere Octene: 3 %) wurden über Natrium am Rückfluß erhitzt und unter Argon destilliert.

Im Autoklaven wurden unter Argonatmosphäre Lösungen der Katalysatorvorstufe und des Liganden gemischt. Als Katalysatorvorstufe kam [(acac)Rh(COD)] (Umicore, acac = Acetylacetonat-Anion; COD = 1,5-Cyclooctadien) zum Einsatz. Von der Rhodiumverbindung wurden 10 ml einer 4,31 millimolaren Lösung in den Autoklaven gegeben. Anschließend wurde die entsprechende Menge an Liganden (Rh/Ligand = 1:5) in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurden die n-Octene (10,70 g; 95,35 mmol) eingefüllt. Der Autoklav wurde bei einem Gesamtgasdruck (Synthesegas: Linde; H₂ (99,999%) : CO (99,997%) = 1:1) von 42 bar aufgeheizt. Nach Erreichen der Reaktionstemperatur von 120 °C wurde der Synthesegasdruck auf 48,5 bar erhöht und das Olefin mit einem in der Druckpipette eingestellten Überdruck von ca. 3 bar in den Autoklaven gepresst. Die Reaktion wurde bei konstantem Druck von 50 bar (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m × 0,2 mm × 0,5 µm.

### Ergebnisse der Katalyseversuche

**Tabelle 1: Hydroformylierung der n-Octene**

| Eintrag | Ligand | Regioselektivität in % |
|---|---|---|
| 1 | **V1** | 84 |
| 2 | **1** | 85 |
| 3 | **2** | 85 |
| 4 | **3*** | 88 |

| | | |
|---|---|---|
| * erfindungsgemäße Verbindung | | |

Definition der Selektivität:
Bei der Hydroformylierung gibt es die n/iso-Selektivitäten: das Verhältnis von linearem Aldehyd (= n) zu verzweigtem Aldehyd (= iso). Hierbei bedeutet die Regioselektivität bezüglich des n-Aldehyden, dass diese Menge an linearem Produkt gebildet wurde. Die restlichen Prozente entsprechen dann dem verzweigten Isomer. Bei einer Regioselektivität von 50% entstehen also n-Aldehyd und iso-Aldehyd zu gleichen Teilen.

Mit der erfindungsgemäßen Verbindung (**3**) konnte eine gegenüber dem Vergleichsliganden (**V1**) gesteigerte Regioselektivität erzielt werden.

Die durchgeführten Versuche belegen, dass die gestellte Aufgabe durch die erfindungsgemäßen Verbindungen gelöst wird.

## Patentansprüche

1. Verbindung der Formel (**I**): 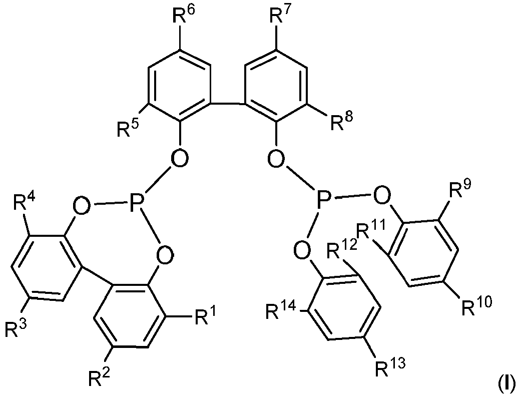 wobei
R¹, R⁴, R⁵, R⁸, R⁹, R¹¹, R¹², R¹⁴ ausgewählt sind aus: -H, -(C₁-C₁₂)-Alkyl, -O-(C₁-C₁₂)-Alkyl, Halogen, -NH₂, -NO₂;
R², R³, R⁶, R⁷ ausgewählt sind aus: -H, -O-(C₁-C₁₂)-Alkyl, -NH₂, -NO₂;
R¹⁰, R¹³ für -NO₂ stehen.

2. Verbindung nach Anspruch 1,
wobei R¹, R⁴, R⁵, R⁸, R⁹, R¹¹, R¹², R¹⁴ ausgewählt sind aus: -H, -(C₁-C₁₂)-Alkyl, -O-(C₁-C₁₂)-Alkyl, -NO₂.

3. Verbindung nach einem der Ansprüche 1 bis 2,
wobei R² und R³ für -O-(C₁-C₁₂)-Alkyl stehen.

4. Verbindung nach einem der Ansprüche 1 bis 3,
wobei R⁶ und R⁷ für -O-(C₁-C₁₂)-Alkyl stehen.

5. Verbindung nach einem der Ansprüche 1 bis 4,
wobei R¹ und R⁴ für -(C₁-C₁₂)-Alkyl stehen.

6. Verbindung nach einem der Ansprüche 1 bis 5,
wobei R⁵ und R⁸ für -(C₁-C₁₂)-Alkyl stehen.

7. Verbindung nach einem der Ansprüche 1 bis 6, wobei R⁹, R¹¹, R¹², R¹⁴ für-H stehen.

8. Verbindung nach einem der Ansprüche 1 bis 7, wobei die Verbindung die Struktur (3) aufweist:

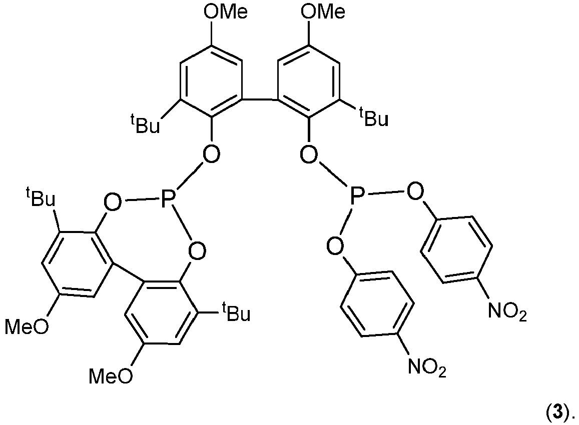

9. Verwendung einer Verbindung nach Anspruch 1 bis 8,
in einem Ligand-Metall-Komplex zur Katalyse einer Hydroformylierungsreaktion.

10. Verfahren umfassend die Verfahrensschritte:
a) Vorlegen eines Olefins,
b) Zugabe einer Verbindung nach einem der Ansprüche 1 bis 8 und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

## Claims

1. Compound of the formula (I): 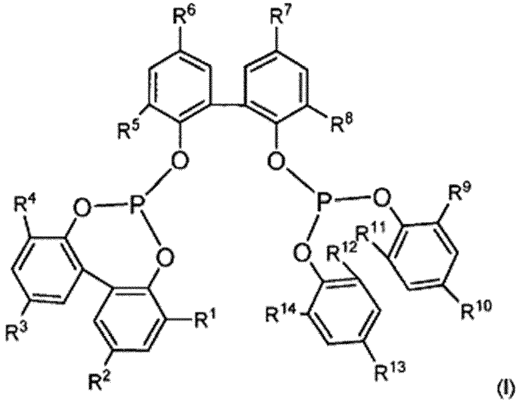 wherein
R¹, R⁴, R⁵, R⁸, R⁹, R¹¹, R¹², R¹⁴ are selected from: -H, - (C₁-C₁₂) -alkyl, -O-(C₁-C₁₂) -alkyl, halogen, -NH₂, -NO₂;
R², R³, R⁶, R⁷ are selected from: -H, -O-(C₁-C₁₂)-alkyl, - NH₂, -NO₂;
R¹⁰, R¹³ are -NO₂.

2. Compound according to Claim 1,
wherein R¹, R⁴, R⁵, R⁸, R⁹, R¹¹, R¹², R¹⁴ are selected from: -H, -(C₁-C₁₂)-alkyl, -O-(C₁-C₁₂)-alkyl, -NO₂.

3. Compound according to either of Claims 1 and 2, wherein R² and R³ are -O-(C₁-C₁₂)-alkyl.

4. Compound according to any of Claims 1 to 3, wherein R⁶ and R⁷ are -O-(C₁-C₁₂) -alkyl.

5. Compound according to any of Claims 1 to 4, wherein R¹ and R⁴ are - (C₁-C₁₂) -alkyl.

6. Compound according to any of Claims 1 to 5, wherein R⁵ and R⁸ are - (C₁-C₁₂) -alkyl.

7. Compound according to any of Claims 1 to 6, wherein R⁹, R¹¹, R¹², R¹⁴ are -H.

8. Compound according to any of Claims 1 to 7, wherein the compound has the structure (3):

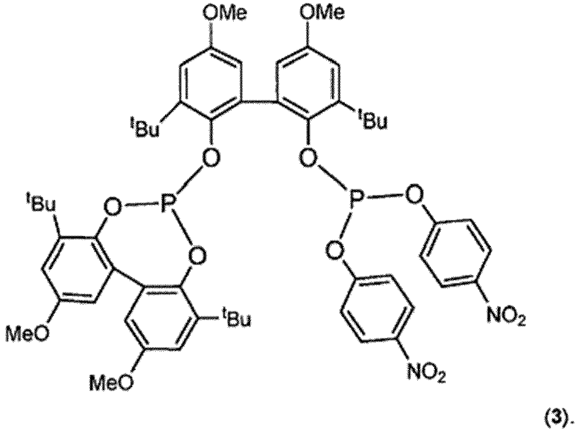

9. Use of a compound according to Claims 1 to 8 in a ligand-metal complex for catalysis of a hydroformylation reaction.

10. Process comprising the process steps of:
a) initially charging an olefin,
b) adding a compound according to any of Claims 1 to 8 and a substance containing a metal selected from: Rh, Ru, Co, Ir,
c) supplying H₂ and CO,
d) heating the reaction mixture of a) to c), with conversion of the olefin to an aldehyde.

## Revendications

1. Composé de formule (I) 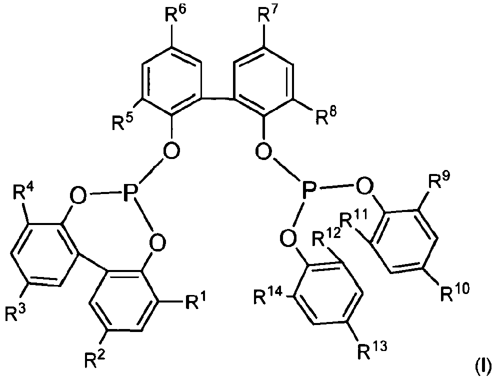 dans laquelle
R¹, R⁴, R⁵, R⁸, R⁹, R¹¹, R¹², R¹⁴ sont choisis parmi -H, un -alkyle en C₁-C₁₂, -O-alkyle en C₁-C₁₂, un halogène, -NH₂, -NO₂ ;
R², R³, R⁶, R⁷ sont choisis parmi: -H, -O-alkyle en C₁-C₁₂, -NH₂, -NO₂;
R¹⁰, R¹³ représentent -NO₂.

2. Composé selon la revendication 1,
dans lequel R¹, R⁴, R⁵, R⁸, R⁹, R¹¹, R¹², R¹⁴ sont choisis parmi -H, -alkyle en C₁-C₁₂, -O-alkyle en C₁-C₁₂, -NO₂.

3. Composé selon l'une des revendications 1 à 2, dans lequel R² et R³ représentent -O-alkyle en C₁-C₁₂.

4. Composé selon l'une des revendications 1 à 3, dans lequel R⁶ et R⁷ représentent -O-alkyle en C₁-C₁₂.

5. Composé selon l'une des revendications 1 à 4, dans lequel R¹ et R⁴ représentent un -alkyle en C₁-C₁₂.

6. Composé selon l'une des revendications 1 à 5, dans lequel R⁵ et R⁸ représentent un -alkyle en C₁-C₁₂.

7. Composé selon l'une des revendications 1 à 6, dans lequel R⁹, R¹¹, R¹², R¹⁴ représentent -H.

8. Composé selon l'une des revendications 1 à 7, le composé présentant la structure (3) :

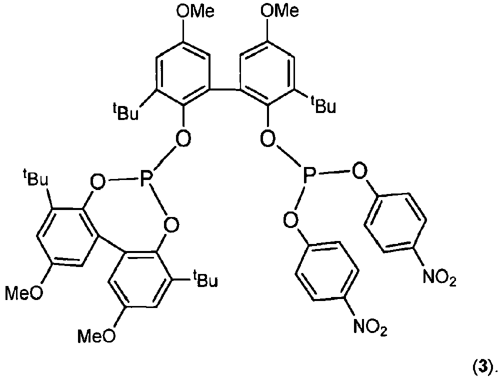

9. Utilisation d'un composé selon les revendications 1 à 8 dans un complexe ligand-métal pour la catalyse d'une réaction d'hydroformylation.

10. Procédé comprenant les étapes suivantes :
a) mise en place d'une oléfine,
b) addition d'un composé selon l'une des revendications 1 à 8 et d'une substance qui comprend un métal choisi parmi Rh, Ro, Cu, Ir,
c) amenée de H₂ et de CO,
d) chauffage du mélange réactionnel de a) à c), l'oléfine étant mise à réagir pour donner un aldéhyde.
